# EUROPÄISCHE PATENTSCHRIFT

(11) **EP 1 343 907 B1**
(45) Veröffentlichungstag und Bekanntmachung des Hinweises auf die Patenterteilung: **30.05.2007**
(21) Anmeldenummer: 01989605.9
(22) Anmeldetag: 19.12.2001
(51) Int. Cl.: C12P 17/12, C07D 239/06

(54) **VERFAHREN ZUR KONTINUIERLICHEN BIOTECHNOLOGISCHEN HERSTELLUNG VON KOMPATIBLEN SOLUTEN AUS TOXISCHEN SUBSTRATEN**
METHOD FOR THE CONTINUOUS BIOTECHNOLOGICAL PRODUCTION OF COMPATIBLE SOLUTES FROM TOXIC SUBSTRATES
PROCEDE DE PRODUCTION BIOTECHNOLOGIQUE CONTINUE DE SOLUTES COMPATIBLES, A PARTIR DE SUBSTRATS TOXIQUES

(30) Priorität: 21.12.2000 DE 10065071
(43) Veröffentlichungstag der Anmeldung: 17.09.2003
(73) Patentinhaber: Helmholtz-Zentrum für Umweltforschung GmbH-UFZ, 04318 Leipzig (DE)
(72) Erfinder: BABEL, Wolfgang, 04329 Leipzig (DE); KLEINSTEUBER, Sabine, 06108 Halle (DE); MASKOW, Thomas, 06688 Grosskorbetha (DE)
(74) Vertreter: Ziebig, Marlene
(86) Internationale Anmeldenummer: PCT/EP2001/014981
(87) Internationale Veröffentlichungsnummer: WO 2002/050298

(56) Entgegenhaltungen:
- DE-A- 19 820 168
- SAUER T ET AL: "BACTERIAL MILKING: A NOVEL BIOPROCESS FOR PRODUCTION OF COMPATIBLE SOLUTES" BIOTECHNOLOGY AND BIOENGINEERING, INTERSCIENCE PUBLISHERS, LONDON, GB, Bd. 57, Nr. 3, 1998, Seiten 306-313, XP000919323 ISSN: 0006-3592 in der Anmeldung erwähnt
- HINTEREGGER C ET AL: "HALOMONAS SP., A MODERATELY HALOPHILIC STRAIN, FOR BIOTREATMENT OF SALINE PHENOLIC WASTE-WATER" BIOTECHNOLOGY LETTERS, KEW, SURREY, GB, Bd. 19, Nr. 11, November 1997 (1997-11), Seiten 1099-1102, XP002938002 ISSN: 0141-5492
- MASKOW T ET AL: "Calorimetrically recognized maximum yield of poly-3-hydroxybutyrate (PHB) continuously synthesized from toxic substrates" JOURNAL OF BIOTECHNOLOGY, ELSEVIER SCIENCE PUBLISHERS, AMSTERDAM, NL, Bd. 77, Nr. 2-3, Februar 2000 (2000-02), Seiten 247-253, XP004185823 ISSN: 0168-1656

## Beschreibung

Die Erfindung betrifft die biotechnologische Synthese von kompatiblen Soluten aus Substraten ausgewählt aus toxischen C1- und C2-Körpern, aliphatischen Kohlenwasserstoffen oder Aromaten oder ökotoxischen Substraten, die potentiell toxisch sind, und die, wenn sie als Kohlenstoff- und Energiequelle für Wachstum und Vermehrung genutzt werden, bereits bei kleinen Konzentrationen das Phänomen der Wachstumsinhibierung (Substratüberschusshemmung) zeigen.

Die Fähigkeit, das osmotische Gleichgewicht zur Umgebung durch die Synthese kompatibler Solute zu erhalten, ist bei Bakterien, Pilzen, Hefen, Algen, Rotalgen oder höheren Organismen weit verbreitet (Brown 1976). Sie ist weder an ein bestimmtes Substrat noch einen bestimmten Ernährungstyp gekoppelt. Kompatible Solute können sowohl aus CO₂ phototroph oder chemolithotroph als auch aus organischen Verbindungen chemoorganoheterotroph gewonnen werden. Neben Zuckern und Alkoholen kommen prinzipiell auch organische Verbindungen wie Kohlenwasserstoffe, organische Säuren oder aromatische Verbindungen in Frage. Zahlreiche Substanzen sind bisher geprüft worden, lediglich Art und Zusammensetzung der synthetisierten kompatiblen Solute hängen vom Organismus, der Kohlenstoffquelle und der Art der Energiebereitstellung ab. Zu den kompatiblen Soluten gehören unterschiedlichste Substanzen (Galinski 1995), darunter Disaccharide, Tetrahydropyrimidincarbonsäuren, Betaine, Glycerol, Glucosylglycerol, Dimethylsulfoniopropionat, Prolin, Nδ-Acetylornithin, Nε-Acetyllysin, Nα-Carbamoylglutaminamide, Nα-Acetylglutaminylglutaminamid.

Wirtschaftliche Verfahren zur biotechnologischen Synthese einiger kompatibler Solute sind bekannt. Die Synthese kompatibler Solute ist häufig an die enzymatischen Leistungen lebender Mikroorganismen gebunden. Im Prinzip existieren für diese Substanzen zwei Syntheseverfahren. Das erste benutzt gentechnisch veränderte Organismen GVO, welche kompatible Solute überproduzieren und in das Medium bis zu Konzentrationen von 0.1 M ausschütten (Grammann et al. 2001). Das zweite Verfahren (Galinski et al. 1992, Sauer und Galinski 1997), das *bacterial milking,* basiert auf der Fähigkeit halophiler Bakterien, Verdünnungsstress durch das schnelle Ausscheiden kompatibler Solute zu bewältigen, um so das Platzen der Zellen zu verhindern. Durch einen zyklischen Wechsel mit einer Synthesephase, ausgelöst durch hohe Salzkonzentrationen, kann man das Verfahren ökonomisch gestalten. In der Synthesephase ist man an hohen Konversionsraten interessiert, die hohe Substratkonzentrationen voraussetzen. Da viele Substanzen, selbst Glucose, das Wachstum bei hohen Konzentrationen hemmen, kann man sich, um trotzdem hohe Zelldichten zu erreichen, mit kontinuierlichen, exponentiell ansteigenden Fütterungsraten behelfen (Keller und Dunn 1978). Substrate, die das Phänomen der Substratüberschussinhibierung bereits bei niedrigen Konzentrationen zeigen, sind ungeeignet.

Bekanntermaßen stellen potentiell toxische Substanzen ein Problem für die Stoffentsorgung dar.

Überraschenderweise wurde nun gefunden, dass kompatible Solute alternativ auch aus solch potentiell toxischen Substanzen hergestellt werden können, welche sind: toxische C1- und C2-Körper, Kohlenwasserstoffe oder auch ökotoxische Substrate wie Phenole oder Benzoate. Erfindungsgemäß wurde ein Prozessregime entwickelt, das die Synthese kompatibler Solute vorzugsweise aus toxischen *Bulkchemicals* oder Abprodukten und Reststoffen der chemischen Industrie und der Landwirtschaft ermöglicht.

Gegenstand der Erfindung ist deshalb ein Verfahren, mit dem kompatible Solute aus toxischen Substraten hergestellt werden können, indem entsprechende halophile Mikroorganismen, die diese Substrate verwerten, kontinuierlich so vermehrt werden, dass die Wärmeproduktion kurz vor einem rapiden Abfall gehalten wird. An diesem Punkt ist der Gehalt an kompatiblen Soluten maximal. Er wird durch geschickte Variation der Zusammensetzung zweier Substratströme bei geringen Volumenaustauschen angesteuert. Für die Synthese in Betracht kommen die halophilen Mikroorganismen, die in bezug auf Nährstoffe anspruchslos sind, schnell wachsen und das gewünschte Produkt in hohen Konzentrationen synthetisieren. Erfindungsgemäß zielt das Verfahren auf die genannten Organika oder Organikagemische, welche cytotoxische Eigenschaften aufweisen und nicht recalzitrant sind.

Das erfindungsgemäße Verfahren zur kontinuierlichen, biotechnologischen Herstellung kompatibler Solute ist demzufolge dadurch gekennzeichnet, dass die halophilen Organismen auf Substraten ausgewählt aus toxischen C1- und C2-Körpern, aliphatischen Kohlenwasserstoffen oder Aromaten oder ökotoxischen Substraten, die Wachstumsinhibition bei Substratüberschuss bereits bei kleinen Konzentrationen zeigen, kontinuierlich vermehrt werden, wobei die Wärmeproduktion kontrolliert und nach Erreichen eines stationären Zustandes im Bereich kurz vor ihrem rapiden, schlagartigen Abfall gehalten wird, indem ein zweiter Sustratstrom zudosiert wird, der den Salzgehalt reguliert, so dass die jeweiligen Solute gebildet werden. Die kontinuierliche Herstellung wird im Temperaturbereich zwischen 10 und 90 °C und bei pH-Werten zwischen 3 und 11 aerob, mikroaerob oder anaerob durchgeführt. Vorzugsweise werden Spezies der Gattung Halomonas auf Phenol, Benzoesäure oder Methanol vermehrt.

Insbesondere werden die Spezies der Gattung Halomonas auf Phenol mit Durchsatzgeschwindigkeiten zwischen 0,07 bis 1,5 g l⁻¹ h⁻¹ und mit spezifischen Wachstumsgeschwindigkeiten zwischen 0,03 und 0,5 h⁻¹ bei einem pH-Wert zwischen 8 und 11 vermehrt. In weiteren Ausführungsvarianten werden die Spezies der Gattung Halomonas auf Bezoesäure mit Durchsatzgeschwindigkeiten zwischen 0,07 bis 3 g l⁻¹ h⁻¹ und mit spezifischen Wachstumsgeschwindigkeiten zwischen 0,03 und 0,5 h⁻¹ oder auf Methanol mit Durchsatzgeschwindigkeiten zwischen 0,07 bis 6,0 g l⁻¹ h⁻¹ und mit spezifischen Wachstumsgeschwindigkeiten zwischen 0,03 und 0,2 h⁻¹ jeweils bei einem pH-Wert zwischen 8 und 11 vermehrt.

In einer besonders bevorzugten Ausführungsform der Erfindung wird ein alkaliphiles Isolat der Gattung *Halomonas* (EF11) bei Phenolverbrauchsgeschwindigkeiten von 0,3 bis 1 gl⁻¹h⁻¹ mit einer Geschwindigkeit zwischen 0,05 und 0,3 h⁻¹ vermehrt. In einer zweiten bevorzugten Ausführungsform wird ein anderes alkaliphiles Isolat der Gattung *Halomonas* (D10) bei Benzoesäureverbrauchsgeschwindigkeiten von 0,3 bis 1,5 g l⁻¹ h⁻¹ mit einer Geschwindigkeit zwischen 0,05 und 0,4 h⁻¹ vermehrt.

Das erfindungsgemäße Verfahren nutzt die kostengünstigen Kohlenstoffquellen ausgewählt aus toxischen C1- und C2-Körpern, Kohlenwasserstoffen, aber auch ökotoxischen Substraten wie Phenole oder Benzoate. Es hat damit den großen Vorteil, dass eine Problemstoffentsorgung bei gleichzeitiger Wertstoffsynthese erfolgt.

Es können diese toxischen Stoffe verwendet werden, die das Phänomen der Substratüberschussinhibierung bereits bei kleinen Konzentrationen zeigen. Eigentlich sind sie im herkömmlichen Sinn für mikrobiologische Verfahren zur Erzeugung von kompatiblen Soluten nicht geeignet, denn wirtschaftliche Raum-Zeitausbeuten lassen sich mit solchen Substanzen weder im Batch noch im Fed-Batch oder in der kontinuierlichen Prozessgestaltung erreichen, da der Widerspruch zwischen hohen Produktsyntheseraten infolge hoher Substratkonzentrationen und Inhibierung derselbigen durch cytotoxische Effekte so nicht gelöst werden kann. Reduzierte C1-Körper, wie z.B. Formaldehyd aber auch Acetaldehyd, Essigsäure, aliphatische Kohlenwasserstoffe oder Aromaten wie Phenole, Phenoxyalkansäuren, Benzoesäure und Benzaldehyd, wobei insbesondere letztere in bezug auf ihre bateriozide Wirkung bekannt sind und häufig nennenswerte Komponenten in Industrieabwässern darstellen, können erfindungsgemäß einfach entsorgt werden.

### Literatur

Brown AD (1976) Microbial Water Stress. Bacteriological Reviews 40: 803-846
Galinski EA (1995) Osmoadaptation in Bacteria. Adv Microbial Physiol 37: 273-328
Galinski EA, Trüper H-G, Sauer T (1992) Verfahren zur in vivo Gewinnung von Inhaltsstoffen aus Zelle. DE 42 44 580 A1
Grammann K, Volke A, Kunte HJ (2001) Identification and Characterization of the Osmoregulated Ectoine Transport System TeaABC in *Halomonas* elongata DSM 2581^{T}. In: Halophiles 2001, Sevilla, Spain, 23.-27.09.2001 Seite L42
Keller R, Dunn IJ (1978) Fed-batch microbial culture: models, errors and applications. J appl Chem Biotechnol 28: 508-514
Sauer T, Galinski EA (1997) Bacterial Milking: A Novel Bioprocess for Production of Compatible Solutes. Biotechnology and Bioengineering 57:306-313

Nachfolgend wird die Erfindung an Ausführungsbeispielen näher erläutert, ohne sie darauf einzuschränken.

### Ausführungsbeispiele:

### Beispiel 1:

Es wird ein Isolat (EF11) aus einem ungarischen Sodasee, das der Gattung Halomonas angehört, verwendet. Die Kultivierung erfolgt kontinuierlich in einem 2,2 1 fassenden thermisch isolierten Bioreaktor bei pH 9,0 und 30°C. Alle in diesem und den weiteren Beispielen verwendeten Medien enthalten 3g KCl, 0, 5 g NH₄NO₃, 0, 3 g (NH₄)₂SO₄, 0,4 g MgSO₄ x 7 H₂O, 0,3 g NaH₂PO₄, 0,033 ml Extran^{®} AP31 Entschäumer, 1,8 mg FeCl₂ x 4 H₂O, 0,25 mg CoCl₂ x 6 H₂O, 0,01 mg NiCl₂ x 6 H₂O, 0,01 mg CuCl₂ x 2 H₂O, 0,07 mg MnCl₂ x 4 H₂O, 0, 1 mg ZnCl₂, 0,5 mg H₃BO₃, 0,03 mg Na₂MoO₄ x 2 H₂O, 0,01 mg Na₂SeO₃ x 5 H₂O 0,02 mg Biotin, 0,02 mg Folinsäure, 0,1 mg Pyridoxin-HCl, 0,05 mg Thiamin-HCl x 2 H₂O, 0,05 mg Riboflavin, 0,05 mg Nikotinsäure, 0,05 mg D-Ca-pantothenat, 0,001 mg Vitamin B₁₂, 0,05 mg p-Aminobenzoesäure, 0,05 mg Liponsäure auf einen Liter destilliertes Wasser. Der Bioreaktor wird mit 1,7 1 Nährmedium mit 150 mg/l Phenol mit 3 (w/v) % befüllt. Ein Kühlmedium entzieht dem Reaktor einen zeitlich konstanten Wärmefluss und die Reaktortemperatur wird mittels eines elektrischen Heizers konstant gehalten. Die Luft zur Begasung wird mittels einer Waschflaschenkaskade mit Wasserdampf bei Reaktortemperatur gesättigt und die Begasungsrate ist 0,1 m³ h⁻¹ (bezogen auf 101, 325 KPa und 25 °C). Die Homogenisierung erfolgt mittels einer Rushton-Turbine mit 900 Umdrehungen pro Minute. Zulaufendes Medium und 1 N NaOH zur Neutralisation werden mittels eines Wärmeaustauschers auf Reaktortemperatur gebracht. 300 ml einer Vorkultur, batchweise auf eine Biomassedichte von ca. 0,5 g/l gezüchtet, dient als Inokolum. Die Heizleistung, die nötig ist, um die Reaktortemperatur konstant zuhalten, verringert sich um die Wärmeproduktion der Mikroorganismen, sobald diese anfangen, das Phenol zu verwerten. Sofort, wenn die Wärmeproduktion abfällt und damit den Verbrauch an Phenol indiziert, wird erneutes Wachstum durch eine 20 ml Portion des Mediums mit 6,3 g/l Phenol initiiert. Durch Wiederholungen des Vorganges wird eine Biomassedichte von ca. 2 g/l erreicht und anschliessend das Medium kontinuierlich mit 0.14 l h⁻¹ zugegeben und ein Reaktorfüllstand von 2 l eingestellt Nach ca. 30 Stunden zeigt eine konstante Wärmeproduktion von ca. 3,6 W das Erreichen eines stationären Zustandes an. Die Phenolrestkonzentration im Bioreaktor ist dann kleiner als 0,1 mg/l. Danach wird der Salzgehalt des Mediums entweder durch die getrennte Zugabe zweier Medien unterschiedlichen Salzgehaltes oder durch ein Mischsystem langsam und kontinuierlich gesteigert. Ein solches Mischsystem kann z.B. aus einer Mischflasche bestehen, welche den Fermenter mit 0,14 l h⁻¹ speist (Salzkonzentration zu Beginn 8.5 (w/v) %) und einer Reservoirflasche (Salzkonzentration 25 (w/v) %), welche wiederum die Mischflasche mit 0,07 l h⁻¹ speist. Die Wärmeproduktion nimmt linear ab, bis ein schlagartig beschleunigter Abfall der Wärmeproduktion auftritt. Dieser Punkt tritt nach 35 h auf. Er ist charakterisiert durch eine Wärmeproduktion von 2,7 W, eine Biomassedichte von 1,5 g/l mit einem Gehalt an Ectoin von 17 % (w/w) und Hydroxyectoin von 1 % (w/w). Zur Stabilisierung wird der Zulauf von der Reservoirflasche gestoppt und 1 Vol % eines salzfreien Mediums in die Mischflasche gegeben.

### Beispiel 2:

Das Isolat (EF11) wird analog Beipiel 1 kontinuierlich vermehrt. Die Fliessgeschwindigkeiten sind jedoch von der Reservoirflasche zur Mischflasche 0,03 l h⁻¹ und von der Mischflasche in den Bioreaktor 0,06 l h⁻¹. Der beschleunigte Abfall der Wärmeproduktion tritt nach ca. 73 h auf. Er ist durch eine Wärmeproduktion von 1,4 W, eine Biomassedichte von 1,7 g/l mit einem Ectoingehalt von 18 % (w/w) und einem Hydroxyectoingehalt von 0,5 % (w/w) charakterisiert.

### Beispiel 3:

Das Isolat (D10) wird analog zu Beispiel 1 kontinuierlich kultiviert. Die Medien enthalten jedoch 10 g/l Natriumbenzoat. Der Knickpunkt in der Wärmeproduktion tritt nach 40 h auf und ist durch eine Wärmeproduktion von 3,0 W eine Biomassedichte von 3,4 g/l mit einem Ectoingehalt von 15 % (w/w) charakterisiert.

## Patentansprüche

1. Verfahren zur kontinuierlichen, biotechnologischen Herstellung kompatibler Solute, **dadurch gekennzeichnet, dass** halophile Mikroorganismen auf Substraten, ausgewählt aus toxischen C₁- und C₂-Körpern, aliphatischen Kohlenwasserstoffen oder Aromaten oder ökotoxischen Substraten, die Wachstumsinhibition bereits bei kleinen Konzentrationen zeigen, kontinuierlich vermehrt werden, wobei die Wärmeproduktion kontrolliert und nach Erreichen eines stationären Zustandes im Bereich kurz vor ihrem rapiden, schlagartigen Abfall gehalten wird, indem ein zweiter Substratstrom zudosiert wird, der den Salzgehalt reguliert, so dass die jeweiligen Solute gebildet werden.

2. Verfahren nach Anspruch 1, **dadurch gekennzeichnet, dass** die kontinuierliche Herstellung im Temperaturbereich zwischen 10 und 90 °C und bei pH-Werten zwischen 3 und 11 aerob, mikroaerob oder anaerob durchgeführt wird.

3. Verfahren nach Anspruch 1 oder 2, **dadurch gekennzeichnet, dass** als Mikroorganismen Bakterien der Gattung Halomonas genutzt werden.

4. Verfahren nach einem der Ansprüche 1 bis 3, **dadurch gekennzeichnet, dass** als toxische Substrate Formaldehyd, Acetaldehyd, Essigsäure, Methanol, Phenole, Phenoxyalkansäuren, Benzoesäure und Benzaldehyd genutzt werden.

5. Verfahren nach Anspruch 3 und 4, **dadurch gekennzeichnet, dass** Spezies der Gattung Halomonas auf Phenol mit Durchsatzgeschwindigkeiten zwischen 0,07 bis 1,5 g l⁻¹ h⁻¹ und mit spezifischen Wachstumsgeschwindigkeiten zwischen 0,03 und 0,5 h⁻¹ bei einem pH-Wert zwischen 8 und 11 vermehrt werden.

6. Verfahren nach Anspruch 5, **dadurch gekennzeichnet, dass** ein alkaliphiles Isolat bei Phenolverbrauchsgeschwindigkeiten von 0,3 bis 1 g l⁻¹ h⁻¹ mit einer Geschwindigkeit zwischen 0,05 und 0,3 h⁻¹ vermehrt wird.

7. Verfahren nach Anspruch 3 und 4, **dadurch gekennzeichnet, dass** Spezies der Gattung Halomonas auf Bezoesäure mit Durchsatzgeschwindigkeiten zwischen 0,07 bis 3 g l⁻¹ h⁻¹ und mit spezifischen Wachstumsgeschwindigkeiten zwischen 0,03 und 0,5 h⁻¹ bei einem pH-Wert zwischen 8 und 11 vermehrt werden.

8. Verfahren nach Anspruch 7, **dadurch gekennzeichnet, dass** ein alkaliphiles Isolat bei Benzoesäureverbrauchsgeschwindigkeiten von 0,3 bis 1,5 g l⁻¹ h⁻¹ mit einer Geschwindigkeit zwischen 0,05 und 0,4 h⁻¹ vermehrt wird.

9. Verfahren nach Anspruch 3 und 4, **dadurch gekennzeichnet, dass** Spezies der Gattung Halomonas auf Methanol mit Durchsatzgeschwindigkeiten zwischen 0,07 bis 6,0 g l⁻¹ h⁻¹ und mit spezifischen Wachstumsgeschwindigkeiten zwischen 0,03 und 0,2 h⁻¹ bei einem pH-wert zwischen 8 und 11 vermehrt werden.

## Claims

1. A method for the continuous biotechnological production of compatible solutes, **characterized in that** halophilic microorganisms are continuously grown on substrates selected from toxic C₁ and C₂ bodies, aliphatic hydrocarbons or aromatic substances or ecotoxic substrates exhibiting growth inhibition even at low concentrations, in which method the production of heat is controlled and, after reaching a steady state, maintained in a region close to rapid, abrupt decrease thereof by metering a second substrate flow which regulates the salt content so that the respective solutes are formed.

2. The method according to claim 1, **characterized in that** continuous production is conducted in a temperature range between 10 and 90°C at pH values between 3 and 11 under aerobic, microaerobic or anaerobic conditions.

3. The method according to claim 1 or 2, **characterized in that** bacteria of the genus Halomonas are utilized as microorganisms.

4. The method according to any of claims 1 to 3, **characterized in that** formaldehyde, acetaldehyde, acetic acid, methanol, phenols, phenoxyalkanoic acids, benzoic acid and benzaldehyde are utilized as toxic substrates.

5. The method according to claims 3 and 4, **characterized in that** species of the genus Halomonas are grown on phenol with flow rates between 0.07 to 1.5 g l⁻¹ h-¹ and with specific growth rates between 0.03 and 0.5 h⁻¹ at a pH value between 8 and 11.

6. The method according to claim 5, **characterized in that** an alkaliphilic isolate is grown at phenol consumption rates of from 0.3 to 1 g l⁻¹ h⁻¹ at a rate between 0.05 and 0.3 h⁻¹.

7. The method according to claims 3 and 4, **characterized in that** species of the genus Halomonas are grown on benzoic acid with flow rates between 0.07 to 3 g l⁻¹ h⁻¹ and with specific growth rates between 0.03 and 0.5 h⁻¹ at a pH value between 8 and 11.

8. The method according to claim 7, **characterized in that** an alkaliphilic isolate is grown at benzoic acid consumption rates of from 0.3 to 1.5 g l⁻¹ h⁻¹ at a rate between 0.05 and 0.4 h⁻¹.

9. The method according to claims 3 and 4, **characterized in that** species of the genus Halomonas are grown on methanol with flow rates between 0.07 to 6.0 g l⁻¹ h⁻¹ and with specific growth rates between 0.03 and 0.2 h⁻¹at a pH value between 8 and 11.

## Revendications

1. Procédé de préparation continue, biotechnologique de solutés compatibles, **caractérisé en ce qu'**on multiplie en continu des micro-organismes halophiles sur des substrats, choisis parmi des substances toxiques en C₁ et C₂, des hydrocarbures aliphatiques ou des aromatiques ou des substrats écotoxiques, qui présentent une inhibition de croissance déjà à des faibles concentrations, moyennant quoi l'on contrôle la production de chaleur et on la maintient après obtention d'un état stationnaire dans une gamme juste avant leur décroissance rapide, brusque, en ajoutant de manière dosée un second courant de substrat, qui régule la teneur en sel, de manière à former les solutés respectifs.

2. Procédé selon la revendication 1, **caractérisé en ce qu'**on effectue la préparation en continu dans une gamme de température entre 10 et 90°C et à un pH entre 3 et 11, en aérobie, micro-aérobie ou anaérobie.

3. Procédé selon la revendication 1 ou 2, **caractérisé en ce qu'**on utilise en tant que microorganismes des bactéries du genre *Halomonas.*

4. Procédé selon l'une des revendications 1 à 3, **caractérisé en ce qu'**on utilise, en tant que substrats toxiques, du formaldéhyde, de l'acétaldéhyde, de l'acide acétique, du méthanol, des phénols, des acides phénoxyalcane, de l'acide benzoïque et du benzaldéhyde.

5. Procédé selon la revendication 3 et 4, **caractérisé en ce qu'**on multiplie des espèces du genre *Halomonas* sur du phénol à des allures de débit entre 0,07 et 1,5 g l⁻¹ h⁻¹ et à des vitesses de croissance spécifiques entre 0,03 et 0,5 h⁻¹, à un pH entre 8 et 11.

6. Procédé selon la revendication 5, **caractérisé en ce qu'**on multiplie un isolat alcalinophile à des vitesses de consommation de phénol de 0,3 à 1 g l⁻¹ h⁻¹, à une vitesse entre 0,05 et 0,3 h⁻¹.

7. Procédé selon la revendication 3 et 4, **caractérisé en ce qu'**on multiplie des espèces du genre *Halomonas* sur de l'acide benzoïque à des allures de débit entre 0,07 et 3 g l⁻¹ h⁻¹ et à des vitesses de croissance spécifiques entre 0,03 et 0,5 h⁻¹, à un pH entre 8 et 11.

8. Procédé selon la revendication 7, **caractérisé en ce qu'**on multiplie un isolat alcalinophile à des vitesses de consommation d'acide benzoïque de 0,3 à 1,5 g l⁻¹ h⁻¹, à une vitesse entre 0,05 et 0,4 h⁻¹.

9. Procédé selon la revendication 3 et 4, **caractérisé en ce qu'**on multiplie des espèces du genre *Halomonas* sur du méthanol à des allures de débit entre 0,07 et 6,0 g l⁻¹ h⁻¹ et à des vitesses de croissance spécifiques entre 0,03 et 0,2 h⁻¹, à un pH entre 8 et 11.
